# EUROPÄISCHE PATENTSCHRIFT

(11) **EP 0 744 936 B1**
(45) Veröffentlichungstag und Bekanntmachung des Hinweises auf die Patenterteilung: **06.06.2001**
(21) Anmeldenummer: 95908918.6
(22) Anmeldetag: 09.02.1995
(51) Int. Cl.: A61K 7/50, A61K 7/06, A61K 7/48

(54) **HAUT- UND HAARBEHANDLUNGSMITTEL**
HAIR CARE PRODUCTS
PRODUITS DE SOINS CAPILLAIRES

(30) Priorität: 18.02.1994 DE 4405127
(43) Veröffentlichungstag der Anmeldung: 04.12.1996
(73) Patentinhaber: Henkel Kommanditgesellschaft auf Aktien, 40191 Düsseldorf (DE)
(72) Erfinder: MÜLLER, Reinhard, D-41812 Erkelenz (DE); SEIDEL, Kurt, D-40589 Düsseldorf (DE); KACZICH, Anke, D-40595 Düsseldorf (DE); HOLLENBERG, Detlef, D-40699 Erkrath (DE); MATZIK, Iduna, D-40699 Erkrath (DE)
(86) Internationale Anmeldenummer: EP9500462
(87) Internationale Veröffentlichungsnummer: WO9522312

(56) Entgegenhaltungen:
- EP-A- 0 502 616
- WO-A-92/06778
- WO-A-95/13863
- DE-A- 4 327 699

## Beschreibung

Die Erfindung betrifft Zubereitungen zur Behandlung von Haut und Haaren mit einer speziellen Wirkstoffkombination.

Die Reinigung und Pflege der Haare ist ein wichtiger Bestandteil der menschlichen Körperpflege. Sowohl die Reinigung der Haare mit Shampoos als auch die dekorative Gestaltung der Frisur beispielsweise durch Färben oder Dauerwellen sind Eingriffe, die die natürliche Struktur und die Eigenschaften der Haare beeinflussen. So können anschließend an eine solche Behandlung beispielsweise die Naß- und Trockenkämmbarkeit des Haares, der Halt und die Fülle des Haares unbefriedigend sein oder die Haare einen erhöhten Spliß aufweisen. Weiterhin ist die gleichmäßige Verteilung der mit Haarfärbemitteln aufgebrachten Farbstoffe häufig problematisch.

Es ist daher seit langem üblich, die Haare einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung beispielsweise Kämmbarkeit, Halt und Fülle der Haare verbessert, die Splißrate verringert oder die Farbverteilung verbessert.

Weiterhin wurden in jüngster Zeit sogenannte Kombinationspräparate entwickelt, um den Aufwand der üblichen mehrstufigen Verfahren, insbesondere bei der direkten Anwendung durch Verbraucher, zu verringern.

Diese Präparate enthalten neben den üblichen Komponenten, beispielsweise zur Reinigung der Haare, zusätzlich Wirkstoffe, die früher den Haarnachbehandlungsmitteln vorbehalten waren. Der Konsument spart somit einen Anwendungsschritt; gleichzeitig wird der Verpackungsaufwand verringert, da ein Produkt weniger gebraucht wird.

Die zur Verfügung stehenden Wirkstoffe sowohl für separate Nachbehandlungsmittel als auch für Kombinationspräparate können noch nicht alle Wünsche der Verbraucher erfüllen. Insbesondere wird weiter nach Wirkstoffen und Wirkstoffkombinationen mit höherer Wirksamkeit bei gleichzeitig guter biologischer Abbaubarkeit gesucht. Außerdem ist die Farberhaltung nach wie vor problematisch, wenn die gefärbten Haare mehrfach mit üblichen Haarwaschmitteln gereinigt werden.

Schließlich kommen zwangsläufig viele Haarbehandlungsmittel mit der Kopfhaut in Berührung. Dies kann, insbesondere bei empfindlichen Personen, zu Irritationen führen. Es besteht daher weiterhin die Aufgabe, die Haarbehandlungsmittel so zu formulieren, daß Irritationen der Kopfhaut möglichst ausgeschlossen werden. Im Idealfall sollten diese Haarbehandlungsmittel sogar eine pflegende Wirkung für die Kopfhaut aufweisen.

Es wurde nun überraschenderweise gefunden, daß Mittel mit einer Kombination dreier bereits für die Haarbehandlung bekannter Wirkstoffklassen die gestellten Anforderungen in hervorragender Weise erfüllen. Insbesondere wird eine sehr gute Naß- und Trockenkämmbarkeit erreicht. Bei colorierten Haaren wird zusätzlich ein starker Egalisierungseffekt unter überraschend hoher Farbschonung erzielt. Weiterhin wurde ein pflegender Effekt für die Kopfhaut beobachtet. Schließlich lassen sich mit dieser Wirkstoffkombination Zubereitungen mit einer sehr guten biologischen Abbaubarkeit fast aller Komponenten formulieren.

Gegenstand der Erfindung ist daher eine wäßrige Zubereitung zur Behandlung von Haut oder Haaren mit üblichen kosmetischen Bestandteilen, dadurch gekennzeichnet, daß sie eine Wirkstoffkombination, bestehend aus
- einem Alkylpolyglykosid der allgemeinen Formel (I)

   RO-(Z)ₓ (I)

   in der R steht für einen Alkylrest mit 6 bis 22 Kohlenstoffatomen, Z für einen Mono- oder Oligosaccharid und x für eine Zahl von 1,1 bis 5, oder deren Anlagerungsprodukten mit 1 bis 10 Molekülen Ethylenoxid und/oder Propylenoxid
- einem Polymeren und
- einem Pflanzenöl, ausgewählt aus Kukuinußöl, (süßem) Mandelöl,
- Walnußöl, Pfirsichkernöl, Avocadoöl, Sojaöl, Sesamöl, Sonnenblumenkernöl, Tsubakiöl, Nachtkerzenöl, Reiskleieöl, Palmkernöl, Mangokernöl, Wiesenschaumkrautöl, Distelöl, Macadamianußöl, Traubenkernöl, Aprikosenkernöl, Babassuöl, Olivenöl, Weizenkeimöl, Kürbiskernöl, Malvenöl, Haselnußöl, Safloröl, Jojobaöl, Canolaöl, Sasanqua-Öl und Shea-Butter
   enthält, mit der Maßgabe, daß die Zubereitung keine der folgenden Zweierkombinationen enthält:
   Süßes Mandelöl / Copolymer aus dem Ester der Methacrylsäure und
   Stearylalkohol + 20 EO und einem oder mehrerer Monomerer, ausgewählt aus Acrylsäure, Methacrylsäure oder deren einfachen Estern,
   Monoiöl / Copolymer aus dem Ester der Methacrylsäure und Stearylalkohol + 20 EO und einem oder mehrerer Monomerer, ausgewählt aus Acrylsäure.
   Methacrylsäure oder deren einfachen Estern,
   Sesamöl / Copolymer aus Acrylamid und neutralisierter 2-Acrylamido-2-methylpropansulfonsäure.

Alle drei Wirkstoffklassen sind dem Fachmann geläufige Bestandteile von Haarbehandlungsmitteln.

Ebenfalls bekannt sind Kombinationen von Alkylglykosiden und Polymeren, beispielsweise aus der DE-OS 32 16 687, der EP-A1-337 354 sowie den deutschen Patentanmeldungen P 42 32 512.9, P 42 32 506.4, P 42 34 413.1 und P 42 34 405.0.

Weiterhin offenbart die EP-A1-502 616 kosmetische Zusammensetzungen, die ein Alkylpolyglykosid-Tensid, ein Wachs sowie gegebenenfalls einen oder mehrere Verdickungsmittel enthalten. Diese Zusammensetzungen können weiterhin als fakultative Bestandteile bestimmte Pflanzenöle sowie Polymere enthalten.

Diesem Stand der Technik sind aber keine Hinweise auf die vorteilhaften Effekte zu entnehmen, die durch die Verwendung der erfindungsgemäßen Wirkstoff-Dreierkombination in Mitteln zur Behandlung von Haut und Haaren, erzielt werden.

Die erste Komponente der erfindungsgemäßen Wirkstoffkombination stellen Alkylpolyglykoside gemäß Formel (I) dar.

Diese Verbindungen gemäß Formel (I) sind durch die folgenden Parameter gekennzeichnet.

Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylglykoside mit x-Werten von 1,1 bis 1,6 sind bevorzugt. Ganz besonders bevorzugt sind Alkylglykoside, bei denen x 1,1 bis 1,4 beträgt.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten. Auch diese Produkte stellen üblicherweise keine einheitlichen Verbindungen dar, sondern weisen in Abhängigkeit von dem gewählten Ethoxylierungsverfahren eine entsprechende Homologenverteilung auf. Solche alkoxylierten Verbindungen können beispielsweise dadurch erhalten werden, daß zur Synthese der Alkylpolyglykoside ethoxylierte Fettalkohole verwendet werden.

Die Alkylpolyglykoside sind in den erfindungsgemäßen Zubereitungen bevorzugt in Mengen von 0,5 bis 20 Gew.-%, bezogen auf die gesamte Zubereitung, enthalten. Mengen von 10 und mehr Gew.-% werden jedoch üblicherweise nur in speziellen Shampoo-Formulierungen eingesetzt. In anderen Mitteln werden üblicherweise Alkylpolyglykosidmengen zwischen 0,5 und 5 Gew.-% besonders bevorzugt.

Die zweite Komponente der erfindungsgemäßen Wirkstoffkombination ist ein Polymer. Es kann sich dabei sowohl um ein kationisches, amphoteres, zwitterionisches, anionisches als auch um ein nichtionogenes Polymeres handeln. Es kann auch vorteilhaft sein, Vertreter verschiedener Polymertypen zusammen einzusetzen.

Die erfindungsgemäß verwendbaren kationischen Polymeren enthalten innerhalb des Polymergerüstes kationische Gruppen. Diese Gruppen können Teil der Polymerkette sein; sie können sich aber auch in Seitenketten befinden, die über Zwischenglieder mit einer Hauptkette verbunden sind. Übliche kationische Gruppen enthalten quartäre Stickstoff- oder Phosphoratome. Gruppen mit quartären Stickstoffatomen sind dabei bevorzugt. Die quartären Stickstoffatome können dabei sowohl 4 unterschiedliche oder z.T. gleiche Substituenten tragen, als auch Teil eines Ringsystems sein. Bevorzugte kationische Gruppen sind Ammonium- und Imidazoliniumgruppen.

Befinden sich die ionischen Gruppen in den Seitenketten, so sind die Polymeren aus Verbindungen aufgebaut, die neben mindestens einer kationischen Gruppe mindestens eine polymerisierbare Gruppe enthalten und frei sind von anionischen Gruppen.

Die polymerisierbare Gruppe ist bevorzugt eine Vinylgruppe. Es sind jedoch auch kationische Polymerisate verwendbar, bei denen die Polymerhauptkette beispielsweise aus Glykosiden aufgebaut ist oder Proteincharakter hat.

Erfindungsgemäß ebenfalls bevorzugt sind kationische Copolymere, die neben den kationischen Monomeren noch mindestens ein nichtionisches Monomer enthalten. Geeignete nichtionische Monomere sind beispielsweise Vinylpyrrolidon, Vinylacetat, Acrylamid, Methacrylamid, Methylacrylat, Ethylacrylat, Methylmethacrylat und Ethylmethacrylat. Vinylpyrrolidon ist ein besonders bevorzugtes nichtionisches Monomer.

Eine Reihe von für die Haarpflege geeigneten kationischen Polymeren sind dem Fachmann bekannt und als Handelsprodukte erhältlich.

Beispiele für solche Polymeren sind:
- quaternierte Cellulose-Derivate, wie sie unter den Bezeichnungen Celquat^{R} und Polymer JR^{R} im Handel erhältlich sind. Die Verbindungen Celquat H 100, Celquat L 200 und Polymer JR^{R}400 sind bevorzugte quaternierte Cellulose-Derivate.
- quaternierte Guar-Derivate, wie sie unter den Bezeichnungen Cosmedia Guar^{R} und Jaguar^{R} im Handel erhältlich sind. Bevorzugte Guar-Derivate sind beispielsweise Cosmedia Guar^{R} C-261 und Jaguar^{R} C 13-S.
- Copolymere des Vinylpyrrolidons mit quaternierten Derivaten des Dialkylaminoacrylats- und -methacrylats, wie beispielsweise mit Diethylsulfat quaternierte Vinylpyrrolidon-Dimethylaminomethacrylat-Copolymere sowie das Vinylpyrrolidon-Methacrylamidopropyltrimethylammoniumchlorid-Copolymere. Solche Verbindungen sind unter den Bezeichnungen Gafquat^{R}734, Gafquat^{R}755 bzw. Gafquat^{R} HS100 im Handel erhältlich.
- Copolymerisate des Vinylpyrrolidons mit Vinylimidazoliummethochlorid, wie sie unter der Bezeichnung Luviquat^{R} angeboten werden.
- Polymere Dimethyldiallylammoniumsalze und deren Copolymere mit Estern und Amiden von Acrylsäure und Methacrylsäure. Die unter den Bezeichnungen Merquat^{R}100 (Poly(dimethyldiallylammoniumchlorid)) und Merquat^{R}550 (Dimethyldiallylammoniumchlorid-Acrylamid-Copolymer) im Handel erhältlichen Produkte sind Beispiele für solche kationischen Polymere.
- Kationisch derivatisierte Silikonöle, wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{R}-Quat 3270 und 7232 (Hersteller: Th. Goldschmidt; diquaternäre Polydimethylsiloxane, Quaternium-80).
- Kationisch derivatisierte Proteinhydrolysate, die beispielsweise durch Umsetzung von alkalisch, sauer oder enzymatisch hydrolysierten Proteinen mit Glycidyltrialkylammoniumsalzen oder 3-Halo-2-hydroxypropyltrialkylammoniumsalzen erhalten werden können, sind im Sinne dieser Erfindung ebenfalls kationische Polymere. Die Proteine, die als Ausgangstoffe für die Proteinhydrolysate dienen, können sowohl tierischer als auch pflanzlicher Herkunft sein. Übliche Ausgangsstoffe sind beispielsweise Keratin, Kollagen, Elastin, Sojaprotein, Milchprotein, Weizenprotein, Seidenprotein und Mandelprotein. Durch die Hydrolyse entstehen Stoffmischungen mit Molmassen im Bereich von ca. 100 bis ca. 50 000 Dalton. Übliche mittlere Molmassen liegen in einem Bereich von etwa 500 bis etwa 5000 Dalton. Nähere Einzelheiten über kationische Derivatisierung können u.a. der japanischen Patentanmeldung 77/73485 (Chemical Abstracts Referat 90:174508v) entnommen werden.
   Vorteilhafterweise enthalten die kationisch derivatisierten Proteinhydrolysate eine oder zwei lange Alkylketten mit 8 bis 22 C-Atomen und entsprechend zwei oder eine kurze Alkylkette mit 1 bis 4 C-Atomen.
   Verbindungen, die eine lange Alkylkette enthalten, sind bevorzugt.
   Bevorzugte Proteinderivate sind Substanzen der Formel (II), 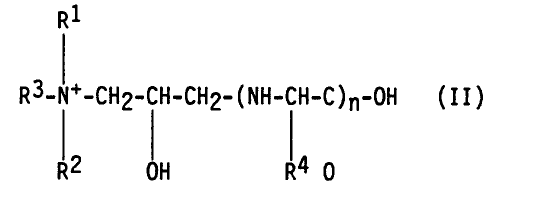 in der R⁴ für die Seitenketten der Aminosäuren des Proteins, R¹ und R² unabhängig voneinander für Alkylketten mit 1 bis 4 C-Atomen und R³ für eine Alkylkette mit 8 bis 22 C-Atomen steht.
   Ein auf dem Markt erhältliches Produkt ist Lamequat^{R}L (Chemische Fabrik Grünau). Es hat die Struktur 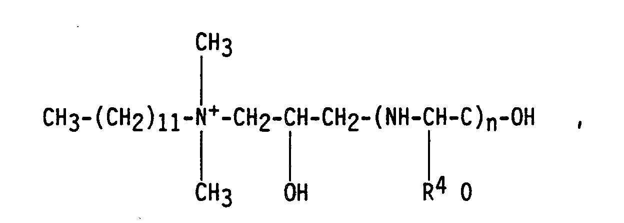 in der R für die Seitenketten der Aminosäuren des Kollagens steht. Eine Bezeichnung analog CTFA ist Lauryldimonium Hydroxypropylamino Hydrolyzed Collagene.
- Polymere Kondensationsharze von Polyolen und Polyaminen, wie beispielsweise Polyglykol-Polyamin-Kondensationsharze, die unter der CTFA-Bezeichnung PEG-15 Cocopolyamine bekannt sind. Im Handel ist beispielsweise das Produkt Polyquart^{R}H 81 (Henkel) erhältlich.

Unter "amphoteren Polymeren" sollen im Sinne der Erfindung Polymere verstanden werden, die im Molekül sowohl freie Aminogruppen als auch freie -C00H- oder -S0₃H-Gruppen enthalten und zur Ausbildung innerer Salze befähigt sind. Unter "zwitterionischen Polymeren" werden solche Polymeren verstanden, die im Molekül quartäre Ammoniumgruppen und -C00⁻- oder -S0₃⁻-Gruppen enthalten.

Beispiele für erfindungsgemäß einsetzbare amphotere Polymere sind die unter den Bezeichnung Amphomer^{R} und Amphomer^{R} LV-71 erhältlichen Acrylharze, die Copolymere aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellen.

Weitere erfindungsgemäß einsetzbare amphotere oder zwitterionische Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Besonders bevorzugt werden zwitterionische Polymere, die sich im wesentlichen zusammensetzen aus
(α) Monomeren mit quartären Ammoniumgruppen der allgemeinen Formel (III),

   R⁵-CH=CR⁶-CO-X-(CₙH₂ₙ)-N⁽⁺⁾R⁷R⁸R⁹ A⁽⁻⁾ (III)

   in der R⁵ und R⁶ unabhängig voneinander stehen für Wasserstoff oder eine Methylgruppe und R⁷, R⁸ und R⁹ unabhängig voneinander für Alkylgruppen mit 1 bis 4 Kohlenstoff-Atomen, X eine NH-Gruppe oder ein Sauerstoffatom, n eine ganze Zahl von 2 bis 5 und A⁽⁻⁾ das Anion einer organischen oder anorganischen Säure ist
   und
(B) monomeren Carbonsäuren der allgemeinen Formel (IV),

   R¹⁰-CH=CR¹¹-COOH (IV),

   in denen R¹⁰ und R¹¹ unabhängig voneinander Wasserstoff oder Methylgruppen sind, oder den Alkali-, Erdalkali-, Aluminium- oder Ammoniumsalzen dieser Säuren.

Bezüglich der Einzelheiten der Herstellung dieser Polymerisate wird ausdrücklich auf den Inhalt der deutschen Offenlegungsschrift 39 29 973 Bezug genommen.

Ganz besonders bevorzugt sind solche Polymeren auf Basis von Monomeren des Typs (α), bei denen R⁷, R⁸ und R⁹ Methylgruppen sind, X eine NH-Gruppe und A⁽⁻⁾ ein Halogenid-, Methoxysulfat- oder Ethoxysulfat-Ion ist; Acrylamidopropyltrimethylammoniumchlorid und Metacrylamidopropyltrimethylammoniumchlorid sind besonders bevorzugte Monomere (α). Als Monomeres (β) für die genannten Polymeren wird bevorzugt Acrylsäure oder ein Alkalisalz der Acrylsäure, insbesondere das Natriumsalz, verwendet.

Weiterhin sind solche zwitterionischen Polymere bevorzugt, bei denen die Zahl der Monomeren vom Typ (α) größer als die Zahl der Monomeren vom Typ (β) ist. Monomerenverhältnisse (α):(β) größer als 1,5 sind besonders bevorzugt.

Ebenfalls bevorzugte zwitterionische Polymerisate sind Polysiloxan-Polyorganobetain-Copolymere sowie zwitterionische Celluloseether gemäß DE-0S-38 33 658.

Erfindungsgemäß geeignete anionische Polymere sind u. a.:
- Vinylacetat/Crotonsäure-Copolymere, wie sie beispielsweise unter den Bezeichnungen Resyn^{R} (National Starch), Luviset^{R} (BASF) und Gafset^{R} (GAF) im Handel sind. Luviset^{R} CA-66 ist ein besonders bevorzugtes anionisches Polymeres.
- Vinylpyrrolidon/Vinylacrylat-Copolymere, erhältlich beispielsweise unter dem Warenzeichen Luviflex^{R} (BASF). Ein bevorzugtes Polymer ist das unter der Bezeichnung Luviflex^{R} VBM-35 (BASF) erhältliche Vinylpyrrolidon/ Acrylat-Terpolymere.
- Vinylacetat/Butylmaleat/Isobornylacrylat-Copolymere, wie sie unter dem Warenzeichen Advantage^{R} (GAF) erhältlich sind. Advantage^{R} CP ist ein bevorzugtes Polymeres.
- Methylvinylether/Maleinsäureanhydrid-Copolymere und deren Ester, wie sie beispielsweise unter der Bezeichnung Gantrez^{R} (GAF) erhältlich sind. Gantrez^{R} ES 225 ist ein bevorzugtes anionisches Polymer.
- Acrylsäure/Ethylacrylat/N-tert.Butylacrylamid-Terpolymere, die beispielsweise unter der Bezeichnung Ultrahold^{R}8 (BASF) vertrieben werden.
- Unvernetzte, teilvernetzte und vernetzte Polyacrylsäuren, Polyacrylsäureester, Acrylsäure-Methacrylsäure-Copolymere und Acrylsäure-Acrylamid-Copolymere, wie sie beispielsweise unter den Bezeichnungen Carbopol^{R}, Latekoll^{R}, Pemulen^{R} und Acrysol^{R} im Handel sind.

Geeignete nichtionogene Polymere sind beispielsweise:
- Polyvinylpyrrolidone, beispielsweise die unter den Bezeichnungen Luviskol^{R} K 30 und Luviskol^{R} K 90 (BASF) erhältlichen Produkte.
- Vinylpyrrolidon/Vinylacetat-Copolymere, wie sie beispielsweise unter dem Warenzeichen Luviskol^{R} (BASF) vertrieben werden. Luviskol^{R} VA 64, Luviskol^{R} VA 73 und Luviskol^{R} VA 37 sind bevorzugte nichtionogene Polymere; Luviskol^{R} VA 37 ist besonders bevorzugt.
- Vinylpyrrolidon/Dimethylaminoethylmethacrylat/Vinylcaprolactam-Terpolymere, wie sie beispielsweise unter der Bezeichnung Copolymer VC-713 (GAF) erhältlich sind.
- Celluloseether, wie beispielsweise Hydroxyethylcellulose, Hydroxypropylcellulose, Methylhydroxypropylcellulose und Hydroxymethylcellulose.

Unter den Polymeren sind die kationischen, zwitterionischen, amphoteren und nichtionogenen Polymeren bevorzugt. Als erfindungsgemäß ganz besonders gut verwendbar haben sich die kationischen und insbesondere die nichtionogenen Polymeren erwiesen.

Die erfindungsgemäßen Zubereitungen enthalten bevorzugt 0,05 bis 5 Gew.%, insbesondere 0,05 bis 2 Gew.-% an Polymeren, bezogen jeweils auf die gesamte Zubereitung.

Die dritte Komponente der erfindungsgemäßen Wirkstoffkombination ist schließlich ein Pflanzenöl. Beispiele für erfindungsgemäß geeignete Pflanzenöle sind Kukuinußöl. (süßes) Mandelöl, Walnußöl, Pfirsichkernöl, Avocadoöl, Sojaöl, Sesamöl, Sonnenblumenkernöl, Tsubakiöl, Nachtkerzenöl. Reiskleieöl, Palmkernöl, Mangokernöl, Wiesenschaumkrautöl, Distelöl, Macadamianußöl, Traubenkernöl, Aprikosenkernöl. Babassuöl. Olivenöl, Weizenkeimöl, Kürbiskernöl, Malvenöl, Haselnußöl. Safloröl. Jojobaöl, Canolaöl, Sasanqua-Öl und Shea-Butter. Unter diesen Ölen sind Sojaöl. Sesamöl. Sonnenblumenkernöl, Wiesenschaumkrautöl, Distelöl, Aprikosenkernöl, Babassuöl. Weizenkeimöl. Jojobaöl, und insbesondere Kukuinußöl, (süßes) Mandelöl, Pfirsichkemöl. Avocadoöl, Nachtkerzenöl, Macadamianußöl und Malvenöl bevorzugt. Als ganz besonders geeignet haben sich Macadamianußöl und insbesondere Kukuinußöl erwiesen.

Die erfindungsgemäßen Zubereitungen können weiterhin alle, für die Art der Zubereitung üblichen kosmetischen Bestandteile enthalten. Hiervon sind insbesondere die verschiedenen Typen von Tensiden zu nennen:

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonatoder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium-, Magnesium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-0-(CH₂-CH₂0)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-0(CH₂-CH₂0)x-0S0₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, sowie Sulfobernsteinsäuremonound -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Als zwitterionische Tenside werden solche oberflächenaktiven Verbindungen bezeichnet, die im Molekül mindestens eine quartäre Ammoniumgruppe und mindestens eine -C00(-)- oder -S0₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈-C₁₈-Alkyl- oder -Acylgruppe im Molekül mindestens eine freie Aminogruppe und mindestens eine -C00H- oder -S0₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Beispiele für in den erfindungsgemäßen Zubereitungen verwendbare kationische Tenside sind insbesondere quartäre Ammoniumverbindungen. Bevorzugt sind Ammoniumhalogenide, insbesondere Chloride und Bromide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können als kationische Tenside sogenannte Esterquats (z. B. Stepantex^{R} VS 90, Dehyquart^{R} AU 36 und AU 56) sowie Amidoamine (z. B. Tegoamid^{R} S 18) eingesetzt werden.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Vorzugsweise enthalten die erfindungsgemäßen Zubereitungen Tenside in Mengen von 0,5 bis 20 Gew.-%, bezogen auf die jeweilige Zubereitung.

Als weitere Lösungsmittel neben Wasser können die erfindungsgemäßen Zubereitungen insbesondere Ethanol und Isopropanol in Mengen bis zu 20 Gew.-%, bezogen auf die gesamte Zubereitung, enthalten. Mengen von 5 bis 10 Gew.-% können besonders bevorzugt sein.

Die erfindungsgemäßen Zubereitungen weisen bevorzugt einen pH-Wert zwischen 2,5 und 7,5, insbesondere zwischen 3,5 und 6,5 auf. Ein pH-Wert von 4,0 bis 5,0 kann in bestimmten Fällen ganz besonders bevorzugt sein. Lediglich bei speziellen Produkten wie Färbemitteln und Dauerwellmitteln können höhere pH-Werte, in der Regel aber nicht oberhalb von 10, bevorzugt sein.

Zur Einstellung dieses pH-Wertes kann praktisch jede für kosmetische Zwecke verwendbare Säure verwendet werden. Üblicherweise, insbesonders wenn es sich bei der Zubereitung nicht um ein Wellmittel handelt, werden Genußsäuren verwendet. Unter Genußsäuren werden solche Säuren verstanden, die im Rahmen der üblichen Nahrungsaufnahme aufgenommen werden und positive Auswirkungen auf den menschlichen Organismus haben. Genußsäuren sind beispielsweise Essigsäure, Milchsäure, Weinsäure, Zitronensäure, Äpfelsäure, Ascorbinsäure und Gluconsäure. Im Rahmen der Erfindung ist die Verwendung von Zitronensäure und Milchsäure besonders bevorzugt.

Weitere übliche Bestandteile der erfindungsgemäßen Zubereitungen können schließlich sein:
- Verdickungsmittel wie Agar-Agar, Guar-Gum, Alginate und Xanthan-Gum,
- Strukturanten wie Glucose und Maleinsäure,
- Proteinhydrolysate, insbesondere Elastin-, Kollagen-, Keratin-, Milcheiweiß-, Sojaprotein- und Weizenproteinhydrolysate sowie deren Kondensationsprodukte mit Fettsäuren
- Parfümöle, Dimethylisosorbid und Cyclodextrine,
- Lösungsvermittler, wie Ethylenglykol, Propylenglykol, Glycerin und Diethylenglykol,
- Farbstoffe,
- Antischuppenwirkstoffe wie Piroctone Olamine und Zink Omadine,
- weitere Substanzen zur Einstellung des pH-Wertes
- Wirkstoffe wie Panthenol, Allantoin, Pyrrolidoncarbonsäuren, Pflanzenextrakte und Vitamine,
- Lichtschutzmittel,
- Konsistenzgeber wie Zuckerester, Polyolester oder Polyolalkylether,
- Fette und Wachse, wie Walrat, Bienenwachs, Montanwachs, Paraffine und Fettalkohole,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat, sowie
- Antioxidantien.

Die erfindungsgemäßen Wirkstoffkombinationen entfalten ihre positiven Wirkungen in allen üblichen Haut- und Haarbehandlungsmitteln. Besonders vorteilhaft ist jedoch ihre Verwendung in Mitteln zur Reinigung und Pflege der Haare eingesetzt. Solche Mittel sind insbesondere Shampoos, Haarspülungen, Haarnachbehandlungsmittel, Haarkuren und Haarfestiger.

Die erfindungsgemäß verwendbaren Mittel können als Lösung, Lotion, Emulsion, Mikroemulsion, Creme oder Gel formuliert sein. Die Formulierung als Lösung, Emulsion oder Mikroemulsion mit einem Wassergehalt von 50 bis 90 Gew.-%, bezogen auf das gesamte Mittel, kann bevorzugt sein.

In einer weiteren bevorzugten Ausführungsform können die Mittel in Form von Schaumaerosolen konfektioniert werden. Dabei ist sowohl die Formulierung mit einem verflüssigten Gas als Treibmittel als auch in Form sogenannter Pumpsprays möglich, bei denen der zum Versprühen benötigte Druck durch mechanisches Pumpen aufgebaut wird. Stickstoff, Luft, Kohlendioxid, Propan, Butan, Isobutan, Pentan und Dimethylether sind bevorzugte Treibmittel. Wenngleich Fluorchlor- und Chlorkohlenwasserstoffe hinsichtlich der erzielten Aerosoleigenschaften exzellente Treibmittel sind, so ist doch ihre Verwendung als Treibmittel in den erfindungsgemäßen Mitteln aufgrund der bekannten Ozon-Problematik weniger bevorzugt.

Gegenstand der Erfindung ist auch die Verwendung der erfindungsgemäßen Zubereitungen zur Behandlung von Haut oder Haaren.

Die erfindungsgemäß verwendeten Zubereitungen können dabei sowohl auf der Haut als auch auf dem Haar verbleiben. Hautcremes, Hautlotionen, Sonnenschutzmittel, Haarnachbehandlungsmittel, Haarkuren und Haarfestiger sind Beispiele für auf diese Weise verwendete Mittel.

Es ist aber ebenfalls möglich, erfindungsgemäße Mittel so zu verwenden, daß die Zubereitung nach einer Einwirkzeit im Bereich von einigen Sekunden bis zu einigen Minuten wieder von der Haut oder dem Haar abgespült wird. Hautreinigungsmittel, Haarshampoos, Haarspülungen und Haarkuren sind Beispiele für solche Mittel.

Die folgenden Beispiele sollen den Erfindungsgegenstand näher erläutern.

### Beispiele

### I. Farbmetrische Untersuchungen zur Farberhaltung bei colorierten Haaren

### Untersuchungsmethode

Eine Egalisiersträhne (naturweiß, 2 g, ca. 15 cm lang; Fa. Kerling) wurde auf halber Höhe abgebunden. Zur Simulation von stark geschädigtem Haar wurde der untere Teil der Strähne abwechselnd je zweimal kaltgewellt und ultrablondiert. Die Kaltwellung erfolgte jeweils durch Behandlung mit einer wäßrigen Lösung eines Kaltwellmittels auf Basis Ammoniumthioglykolat (30 Minuten) und anschließende Fixierung mit Kaliumbromat-Lösung (10 Minuten). Die Ultrablondierung erfolgt mit einer wäßrigen Lösung von Wasserstoffperoxid und Ammoniumperoxidisulfat. Der obere Teil wurde zur Simulation von wenig geschädigtem Haar lediglich einmal ultrablondiert. Anschließend wurde die gesamte Strähne mit dem Handelsprodukt Poly Diadem Pflege Intensiv Tönung (Nuance Mahagoni-Koralle) (HENKEL) ausgefärbt. Dabei wurden 4 g Färbemischung pro 1 g Haarsträhne eingesetzt. Nach einer Einwirkzeit von 30 Minuten wurden die Strähne mit warmem Wasser (30 °C) gut ausgespült und mit einem Fön getrocknet. Die ausgefärbte Haarsträhne wurde dann 2 Tage bei Raumtemperatur gelagert.

Nach der farbmetrischen Bestimmung des Nullwertes wurde die handtuchtrockene Haarsträhne bei abzuspülenden Mitteln 2 Minuten mit 0,3 g Testmischung pro 1 g Haar behandelt. Anschließend wurde die Strähne mit Wasser (30 °C) gründlich gespült, getrocknet und farbmetrisch vermessen. Vor der nächsten Anwendung wurde die Strähne jeweils mit einem marktüblichen Shampoo gewaschen und getrocknet.

Bei auf dem Haar verbleibenden Mitteln wurde auf die handtuchtrockene Haarsträhne 0,3 g Testmischung pro 1 g Haar aufgetragen; danach wurde die Strähne einmal durchgekämmt und über Nacht zum Trocknen belassen. Am nächsten Tag wurde die Strähne dann farbmetrisch vermessen. Vor der nächsten Anwendung wurde die Strähne jeweils mit einem marktüblichen Shampoo gewaschen und getrocknet.

Die Messung wurde mit einem Gerät Texflash (Fa. Datacolor) mit Lichtart D65 (Tageslicht) durchgeführt. Dabei wurde die zu vermessende Probe in einer Einspannvorrichtung am Spektralphotometer fixiert und die Remissionswerte über den Bereich des sichtbaren Lichtes von 390 - 700 nm im Abstand von 10 nm gemessen und über einen Rechner verarbeitet. Das Rechnerprogramm ermittelte die Normfarbwerte nach dem CIE-System (Commission Internationale de L'Eclairage) entsprechend DIN 5033 und rechnete sie in Farbabstandszahlen nach DIN 6174 um. Die angegebenen Werte für den Gesamtfarbabstand DE (relativ zum Nullwert) sind jeweils Mittelwerte aus 4 Meßpunkten pro Strähnenteil.

Es wurden die folgende erfindungsgemäße Mischung und die entsprechende Vergleichsmischung untersucht:
B1 / V1: Schaumkur (verbleibt auf dem Haar)

Die Zusammensetzungen der untersuchten Mischungen und die Meßergebnisse sind in der Tabelle 1 aufgeführt.

Alle Mengenangaben sind Gewichts-%. Bei den Angaben zum Gesamtfarbabstand DE bedeutet N die Zahl der Anwendungen des Mittels und SG der Schädigungsgrad des Haares.

**Tabelle 1:**

| Komponente | B1 | V1 |
|---|---|---|
| Stenol^{R}1618¹ | - | - |
| Tegoamid^{R}S 18² | - | - |
| Cutina^{R}CP³ | - | - |
| Ajidew^{R}N 50⁴ | - | - |
| Citronensäure | - | - |
| Diisopropyladipat | 0,8 | 0,8 |
| Cremophor^{R}RH 40⁵ | 0,1 | 0,1 |
| Luviskol^{R}K 90⁶ | 0,1 | 0,1 |
| Celquat^{R}L 200⁷ | 0,5 | 0,5 |
| Sepigel^{R}305⁸ | 1,0 | 1,0 |
| Dow Corning^{R}345⁹ | 0,2 | 0,2 |
| Plantaren^{R}1200¹⁰ | 1,0 | 1,0 |
| Kukiinußöl | 0,2 | - |
| Ethanol | - | - |
| Wasser | <-----ad 100-----> | |
| | | |
| DE (n/SG) | | |
| - 1 / stark | 1,5 | 2,7 |
| - 3 / stark | 7,8 | 11,0 |
| - 1 / gering | 0,5 | 0,7 |
| - 3 / gering | 4,8 | 5,2 |

| | | |
|---|---|---|
| ¹ C₁₆/C₁₈-Fettalkohol (HENKEL) | | |
| ² N,N-Dimethyl-N'-stearoyl-1,3-diamino-propan (CTFA-Bezeichnung: Stearamidopropyl Dimethylamin) (GOLDSCHMIDT) | | |
| ³ Ester aus gesättigten, langkettigen Fettalkoholen und Fettsäuren, vornehmlich Palmitinsäurecetylester (CTFA-Bezeichnung: Cetyl Palmitate) (HENKEL) | | |
| ⁴ DL-2-Pyrrolidon-5-carbonsäure-Natriumsalz (ca. 50 % Aktivsubstanz; CTFA-Bezeichnung: Sodium PCA) (AJINOMOTO) | | |
| ⁵ Rizinus-Öl, hydriert + 45 Ethylenoxid (CTFA-Bezeichnung: PEG-40 Hydro-genated Castor Oil) (BASF) | | |
| ⁶ Polyvinylpyrrolidon (BASF) | | |
| ⁷ Hydroxyethylcellulose/Diallyldimethylammoniumchlorid (95 % Aktivsubstanz; CTFA-Bezeichnung: Polyquaternium-4) (DELFT NATIONAL) | | |
| ⁸ Dodecylalkohol+7E0-Isoparaffin-Polacrylamid-Mischung (CTFA-Bezeichnung:Polyacrylamide (and) C13-14-Isoparaffin (and) Laureth-7) (SEPPIC) | | |
| ⁹ Dimethylcyclosiloxan-Pentamer (CTFA-Bezeichnung: Cyclomethicone) (DOW CORNING) | | |
| ¹⁰ C₁₂-C₁₆-Alkylglucosid mit Oligomerisationsgrad 1,4 (ca. 50 % Aktivsubstanz; CTFA-Bezeichnung: Lauryl Polyglycosid) (HENKEL) | | |

## Patentansprüche

1. Wäßrige Zubereitung zur Behandlung von Haut oder Haaren mit üblichen kosmetischen Bestandteilen, dadurch gekennzeichnet, daß sie eine Wirkstoffkombination, bestehend aus
- einem Alkylpolyglykosid der allgemeinen Formel (I),
RO-(Z)ₓ (I)
in der R steht für einen Alkylrest mit 6 bis 22 Kohlenstoffatomen, Z für einen Mono- oder Oligosaccharid und x für eine Zahl von 1,1 bis 5, oder deren Anlagerungsprodukten mit 1 bis 10 Molekülen Ethylenoxid und/oder Propylenoxid,
- einem Polymeren und
- einem Pflanzenöl, ausgewählt aus Kukuinußöl, (süßem) Mandelöl, Walnußöl,
Pfirsichkernöl, Avocadoöl, Sojaöl, Sesamöl, Sonnenblumenkernöl, Tsubakiöl, Nachtkerzenöl, Reiskleieöl, Palmkernöl, Mangokernöl, Wiesenschaumkrautöl, Distelöl, Macadamianußöl, Traubenkernöl, Aprikosenkernöl, Babassuöl, Olivenöl, Weizenkeimöl, Kürbiskernöl, Malvenöl, Haselnußöl, Safloröl, Jojobaöl, Canolaöl, Sasanqua-Öl und Shea-Butter enthält, mit der Maßgabe, daß die Zubereitung keine der folgenden Zweierkombinationen enthält:
Süßes Mandelöl / Copolymer aus dem Ester der Methacrylsäure und
Stearylalkohol + 20 EO und einem oder mehrerer Monomerer, ausgewählt aus Acrylsäure, Methacrylsäure oder deren einfachen Estern,
Monoiöl / Copolymer aus dem Ester der Methacrylsäure und Stearylalkohol + 20 EO und einem oder mehrerer Monomerer, ausgewählt aus Acrylsäure, Methacrylsäure oder deren einfachen Estern,
Sesamöl / Copolymer aus Acrylamid und neutralisierter 2-Acrylamido-2-methylpropansulfonsäure.

2. Zubereitung nach Anspruch 1, dadurch gekennzeichnet, daß in der Formel (I) Z für Glucose, R für einen Alkylrest mit 8 bis 18 Kohlenstoffatomen und x für eine Zahl zwischen 1,1 und 1,6, insbesondere für eine Zahl zwischen 1,1 und 1,4, steht.

3. Zubereitung nach Anspruch 1 oder 2, dadurch gekennzeichnet, daß das Polymer ausgewählt ist aus der Gruppe der kationischen, zwitterionischen, amphoteren und nichtionogenen Polymeren.

4. Zubereitung nach Anspruch 3, dadurch gekennzeichnet, daß das Polymer ausgewählt ist aus der Gruppe der nichtionogenen Polymeren.

5. Zubereitung nach einem der Ansprüche 1 bis 4, dadurch gekennzeichnet, daß das Pflanzenöl ausgewählt ist aus Kukuinußöl, Mandelöl, Pfirsichkernöl, Avocadoöl, Nachtkerzenöl, Macadamianußöl und Malvenöl.

6. Zubereitung nach Anspruch 5, dadurch gekennzeichnet, daß das Pflanzenöl Kukuinußöl oder Macadamianußöl ist.

7. Zubereitung nach einem der Ansprüche 1 bis 6, dadurch gekennzeichnet, daß sie
- 0,5 bis 20 Gew.-% Alkylpolyglykosid,
- 0,05 bis 5 Gew.-% Polymer und
- 0,1 bis 5 Gew.-% Pflanzenöl enthält

8. Zubereitung nach einem der Ansprüche 1 bis 7, dadurch gekennzeichnet, daß sie als Schaum-Aerosol formuliert ist.

9. Zubereitung nach Anspruch 8, dadurch gekennzeichnet, daß das Treibgas ausgewählt ist aus Stickstoff, Luft, Kohlendioxid, Propan, Butan, Isobutan, Pentan und Dimethylether.

10. Verwendung einer Zubereitung nach einem der Ansprüche 1 bis 9, zur Behandlung von Haut oder Haaren, dadurch gekennzeichnet, daß die Zubereitung auf der Haut oder dem Haar verbleibt.

11. Verwendung einer Zubereitung nach einem der Ansprüche 1 bis 9, zur Behandlung von Haut oder Haaren, dadurch gekennzeichnet, daß die Zubereitung nach einer Einwirkzeit im Bereich von einigen Sekunden bis zu einigen Minuten wieder von der Haut oder dem Haar abgespült wird.

## Claims

1. A water-based preparation for the treatment of skin or hair containing typical cosmetic components, characterized in that it contains an active-substance combination consisting of
- an alkyl polyglycoside corresponding to general formula (I):
RO-(Z)ₓ (I)
in which R is an alkyl group containing 6 to 22 carbon atoms,
Z is a mono- or oligosaccharide and
x is a number of 1.1 to 5,
or adducts thereof with 1 to 10 molecules of ethylene oxide and/or propylene oxide,
- a polymer and
- a vegetable oil selected from kukui nut oil, (sweet) almond oil, walnut oil, peach kernel oil, avocado oil, soya oil, sesame oil, sunflower seed oil, tsubaki oil, evening primrose oil, rice bran oil, palm kernel oil, mango kernel oil, meadow foam oil, thistle oil, macadamia nut oil, grape seed oil, apricot kernel oil, babassu oil, olive oil, wheat germ oil, pumpkin seed oil, mallow oil, hazel nut oil, safflower oil, jojoba oil, canola oil, sasanqua oil and shea butter,
with the proviso that the preparation does not contain any of the following two-component combinations:
sweet almond oil/copolymer of the ester of methacrylic acid and stearyl alcohol + 20 EO and one or more monomers selected from acrylic acid, methacrylic acid or simple esters thereof,
monoi oil/copolymer of the ester of methacrylic acid and stearyl alcohol + 20 EO and one or more monomers monomers selected from acrylic acid, methacrylic acid or simple esters thereof,
sesame oil/copolymer of acrylamide and neutralized 2-acrylamido-2-methylpropanesulfonic acid.

2. A preparation as claimed in claim 1, characterized in that, in formula (I), Z is glucose, R is an alkyl radical containing 8 to 18 carbon atoms and x is a number of 1.1 to 1.6 and, more particularly, a number of 1.1 to 1.4.

3. A preparation as claimed in claim 1 or 2, characterized in that the polymer is selected from the group of cationic, zwitterionic, amphoteric and nonionic polymers.

4. A preparation as claimed in claim 5, characterized in that the polymer is selected from the group of nonionic polymers.

5. A preparation as claimed in any of claims 1 to 4, characterized in that the vegetable oil is selected fromkukui nut oil, almond oil, peach kernel oil, avocado oil, evening primrose oil, macadamia nut oil and mallow oil.

6. A preparation as claimed in claim 5, characterized in that the vegetable oil is kukui nut oil or macadamia nut oil.

7. A preparation as claimed in any of claims 1 to 6, characterized in that it contains
- 0.5 to 20% by weight of alkyl polyglycoside,
- 0.05 to 5% by weight of polymer and
- 0.1 to 5% by weight of vegetable oil.

8. A preparation as claimed in any of claims 1 to 7, characterized in that it is formulated as a foam aerosol.

9. A preparation as claimed in claim 8, characterized in that the propellent gas is selected from nitrogen, air, carbon dioxide, propane, butane, isobutane, pentane and dimethyl ether.

10. The use of the preparation claimed in any of claims 1 to 9 for the treatment of hair or skin, characterized in that the preparation remains on the hair or skin.

11. The use of the preparation claimed in any of claims 1 to 9 for the treatment of hair or skin, characterized in that the preparation is rinsed off the hair or skin after a contact time of a few seconds to a few minutes.

## Revendications

1. Préparation aqueuse pour le traitement de la peau et des cheveux avec des constituants cosmétiques usuels,
caractérisée en ce qu'
elle contient une combinaison de principes actifs consistant en :
- un alkylpolyglycoside de formule générale (I)
RO-(Z)ₓ (I)
dans laquelle R représente un radical alkyle ayant de 6 à 22 atomes de carbone, Z représente un mono- ou un oligosaccharide et x représente un nombre allant de 1,1 à 5, ou leurs produits d'addition avec de 1 à 10 molécules d'oxyde d'éthylène et/ou d'oxyde de propylène,
- un polymère et
- une huile végétale choisie parmi l'huile de noix de kukui, l'huile d'amandes (douces) et l'huile de noix, l'huile de noyau de pêche, l'huile d'avocat, l'huile de soja, l'huile de sésame, l'huile de noyau de tournesol, l'huile de tsubaki, l'huile d'onagre, l'huile de son de riz, l'huile de palmiste, l'huile de noyau de mangue, l'huile de trèfle, l'huile de chardon, l'huile de noix de Macadamia, l'huile de pépins de raisin, l'huile de noyau d'abricot, l'huile de Babassu, l'huile d'olives, l'huile de germe de blé, l'huile de noyau de calebasse, l'huile de mauve, l'huile de noisette, l'huile de safran, l'huile de jojoba, l'huile de canola, l'huile de camélia sasanqua, et de beurre de carité (shea-butter),
avec la restriction que la préparation ne contient aucune des combinaisons à deux composants :
huile d'amandes douces/copolymère à base d'ester d'acide méthacrylique et alcool stéarique + 20 OE et un ou plusieurs monomères choisis parmi l'acide acrylique, l'acide méthacrylique ou leurs esters simples, huile de monoï/copolymère à base d'ester d'acide acrylique et d'alcool stéarique + 20 0E et un ou plusieurs monomères choisis parmi l'acide acrylique, l'acide méthacrylique ou leurs esters simples, huile de sésame/copolymère à base d'acrylamide et d'acide 2-acrylamido-2-méthylpropane sulfonique neutralisé.

2. Préparation selon la revendication 1,
caractérisée en ce que
dans la formule (I) Z représente du glucose, R représente un radical alkyle ayant de 8 à 18 atomes de carbone, et x représente un nombre compris entre 1,1 et 1,6, en particulier un nombre compris entre 1,1 et 1,4.

3. Préparation selon la revendication 1 ou la revendication 2,
caractérisée en ce que
le polymère est choisi dans le groupe des polymères cationiques, zwitterioniques, amphotères et non ionogènes.

4. Préparation selon la revendication 3,
caractérisée en ce que
le polymère est choisi dans le groupe des polymères non ionogènes.

5. Préparation selon l'une des revendications 1 à 4,
caractérisée en ce que
l'huile végétale est choisie parmi l'huile de noix de kukui, l'huile d'amandes, l'huile de noyau de pêche, l'huile d'avocat, l'huile d'onagre, l'huile de noix de Macadamia et l'huile de mauve.

6. Préparation selon la revendication 5,
caractérisée en ce que
l'huile végétale est l'huile de noix de Kukui ou l'huile de noix de Macadamia.

7. Préparation selon l'une quelconque des revendications 1 à 6,
caractérisée en ce qu'
elle contient :
- de 0,5 à 20 % en poids d'alkylpolyglycoside,
- de 0,05 à 5 % en poids de polymère et
- de 0,1 à 5 % en poids d'huile végétale.

8. Préparation selon l'une quelconque des revendications 1 à 7,
caractérisée en ce qu'
elle est formulée sous forme d'aérosol en mousse.

9. Préparation selon la revendication 8,
caractérisée en ce que
le gaz propulseur est choisi parmi l'azote, l'air, le dioxyde de carbone, le propane, le butane, l'isobutane, le pentane et l'éther diméthylique.

10. Utilisation d'une préparation selon l'une quelconque des revendications 1 à 9 en vue du traitement de la peau ou des cheveux,
caractérisée en ce que
la préparation demeure sur la peau ou sur les cheveux.

11. Utilisation d'une préparation selon l'une des revendications 1 à 9 en vue du traitement de la peau ou des cheveux,
caractérisée en ce que
la préparation après un temps d'action dans la zone de quelques secondes à jusqu'à quelques minutes, est éliminée par rinçage à nouveau, de la peau ou des cheveux.
